# EUROPEAN PATENT SPECIFICATION

(11) **EP 3 073 901 B1**
(45) Date of publication and mention of the grant of the patent: **13.09.2017**
(21) Application number: 14805840.7
(22) Date of filing: 27.11.2014
(51) Int. Cl.: A61B 5/00, A61B 5/11, A61B 5/024, A61B 5/08, A61B 5/113

(54) **SLEEP MONITORING DEVICE AND METHOD**
VORRICHTUNG UND VERFAHREN ZUR SCHLAFÜBERWACHUNG
DISPOSITIF ET PROCÉDÉ DE SURVEILLANCE DU SOMMEIL

(30) Priority: 28.11.2013 EP 13194857
(43) Date of publication of application: 05.10.2016
(73) Proprietor: Koninklijke Philips N.V., 5656 AE Eindhoven (NL)
(72) Inventor: RAYMANN, Roy Joan Eli Marie, 5656 AE Eindhoven (NL); ZWARTKRUIS-PELGRIM, Petronella Hendrika, 5656 AE Eindhoven (NL); BEREZHNYY, Igor, 5656 AE Eindhoven (NL)
(74) Representative: Freeke, Arnold
(86) International application number: PCT/EP2014/075720
(87) International publication number: WO 2015/078937

(56) References cited:
- WO-A2-2007/143535
- US-A- 5 479 932
- US-B1- 6 280 392

## Description

### FIELD OF THE INVENTION

The invention relates to a sleep monitoring device. The invention further relates to a method of monitoring sleep and a corresponding computer program.

### BACKGROUND

Healthy sleep is of crucial importance during the first months of newborn development. Accordingly, devices have been proposed that allow parents or other care givers to monitor the sleep of infants, babies and neonates. In the past this concern was especially related to the so-called sudden infant death syndrome (SIDS) or infant apnea syndrome.

An example of a known monitoring device is given by US patent 6280392 B1, with title "Infant condition monitoring system and method using load cell sensor sheet".

The known infant condition monitoring system includes a sensor sheet placed on a baby bed. The sensor sheet has plural pressure sensitive cells disposed at equal intervals therein, and is placed underneath a mattress on the bed floor. A control unit determines an infant's breathing and sleeping posture from the digital signals obtained from the sensor sheet.

The control unit forms the infant's breathing signal based on changes in distribution of loads which is caused by up-down movements of a diaphragm responsive to the infant's breathing. The load signal from each pressure sensitive cell is subjected to filtering through a band pass filter having a pass band at around a specified frequency corresponding to breathing. If the infant is in the apnea or breathless condition or in the abnormal breathing condition the breathing signal which corresponds to the number of respiration will not be formed. Thus, the infant's breathing condition can be determined to be abnormal.

The control unit also determines the sleeping posture of the infant from the load signals.

A monitoring unit worn by a care provider receives the infant's breathing, sleeping posture, and respiration rate from the control unit. A display displays the respiration rate, and sleeping posture. Thus it is enabled to monitor the infant's condition through the displayed respiration rate and sleeping posture on the display at a place away from the infant. If necessary, the care provider can responsively take preventive measures or emergency or first-aid actions.

Another example of a sleep monitor device is known from WO2007/143535 A2, which shows a movement data analyzer, a data classifier and a sleep classifier. Cardiac and respiratory motion data are used in the assessment of sleep state of a human.

### SUMMARY OF THE INVENTION

It would be advantageous to have an improved sleep monitor device.

A sleep monitoring device for monitoring sleep states of a human is provided. The sleep monitoring device is configured to receive movement data of the resting human from a movement measurement device. The sleep monitoring device comprises a movement data analyzer configured to compute from the movement data at least: heart beat data and respiration data, a data classifier configured to determine a heart rate regularity valuation of the heart beat data within a time interval, and to determine a respiration regularity valuation of the respiration data within the time interval, a sleep classifier configured to obtain a sleep state for the time interval from at least the respiration regularity valuation and the heart rate regularity valuation.

Parents of newborns want to be reassured that the development of their babies is in line with the textbook developmental stages. Moreover, they also want to be reassured that the baby is sleeping well. By classifying the heart rate regularity, and respiration regularity and optionally motion quantity within a time interval a sleep state for the time interval will be obtained. The known baby monitor is focused only on the detection of SIDS, which is unrelated to determining a sleep state. In an embodiment, users, e.g. parents, can track and compare day-night rhythm, sleep content and developmental rest-activity state. In an embodiment, the human is an infant. Furthermore, the movement data analyzer is configured to compute from the movement data at least: heart beat data and respiration data, and physical activity. For example, the movement data analyzer is further configured to compute from the movement data gross movement data, and wherein the data classifier is configured to determine a gross movement extent indicating an extent of the gross movement of the human within the time interval. In another example, the movement data analyzer is further configured to compute from the movement data fine movement data, and wherein the data classifier is configured to determine a fine movement extent indicating an extent of the fine movement of the human within the time interval.

Classifying in gross and fine movement may be done by first obtaining a movement signal, e.g., by subtracting a heart rate and respiration signal from the movement data. The data classifier configured to determine type of movement by quantification of frequency of gross movements, characterized by a high amplitude in movement signal, and frequency of fine movements, characterized by a low amplitude in movement signal.

Instead of heart rate regularity one may equally refer to heart rate variability. In an embodiment, heart rate regularity valuation, respiration regularity valuation, gross movement extent, fine movement extent and vocalization extent are each expressed as a value, e.g. a real number, or an integer. In an embodiment, they have a limited number of value, indication presence, absence or unknown. e.g., indicated as: +1 for presence, -1 for absence, and 0 for unknown.

Distinguishing between gross and fine movement is especially suited for use with a feature vector. For example, in an embodiment, the total amount of energy in the movement data is computed. If the total amount is over first threshold, then gross movement is present, if the total amount is over a second threshold but not over the first, then fine movement is present. If the total amount of energy is below the second threshold then neither fine nor gross movement is present. Instead of total energy, one may also use largest amplitude, or largest amplitude difference.

In an embodiment, the sleep classifier stores data that characterize a sleep state and matches the classified data obtained from the data classifier with the characterizing data. For example, the level of the match may be represented in a match value for the sleep state. The match value may be interpreted as how well a known sleep state may be recognized in the behavior of the infant on the mattress.

In an embodiment, the extraction of multiple parameter from movement data is a dichotomously feature classification. For example, a vector indicating the presence or absence of a feature in a time window. From the vector sleep stage can be derived. For example, the elements of the feature vector may be: regular heart rate yes/no, regular respiration yes/no, etc. The match between this feature vector and stored is sleep stage characteristics is calculated.

In an embodiment, the sleep classifier is configured to determine a sleep state by calculating an inner product between the classified data vector and a classifying vector associated with the sleep state, the classified data vector containing classified information determined from the movement data, e.g., heart rate regularity valuation, the respiration regularity valuation, and preferably, gross and fine movement extent and/or preferably, vocalization extent, etc.

In an embodiment, the classifying vector comprises both feature values that are restricted to a few values and continues values.

In an embodiment, the movement measurement device comprises a load cell for measuring pressure of the human on a mattress on which the human rests. For example, the load cell may use one or more miniature sensors in the bed, e.g., piezo touch plates. Using a load cell allows the measurement of movement and of weight.

In an embodiment, a development classifier is configured to obtain a development state for the human from at least the logged movement data, sleep state data and weight data.

The sleep monitor is an electronic device. Display of the logged data, sleep states, development states etc may be done on a computer, e.g., a mobile electronic device, e.g., mobile phone, smart wearables (e.g. watch/glasses). The sleep monitoring device may be comprised in a development tracker.

An aspect of the invention concerns a sleep monitoring system comprising the sleep monitoring device, a mattress and the movement measurement device arranged with respect to the mattress to measure movement data representing movement of a resting human on the mattress, in particular a baby.

A method according to the invention may be implemented on a computer as a computer implemented method, or in dedicated hardware, or in a combination of both. Executable code for a method according to the invention may be stored on a computer program product. Examples of computer program products include memory devices, optical storage devices, integrated circuits, servers, online software, etc. Preferably, the computer program product comprises non-transitory program code means stored on a computer readable medium for performing a method according to the invention when said program product is executed on a computer.

In a preferred embodiment, the computer program comprises computer program code means adapted to perform all the steps of a method according to the invention when the computer program is run on a computer. Preferably, the computer program is embodied on a computer readable medium.

### BRIEF DESCRIPTION OF THE DRAWINGS

These and other aspects of the invention are apparent from and will be elucidated with reference to the embodiments described hereinafter. In the drawings,
Figure 1 is schematic block diagram of a sleep monitoring system 100,
Figure 2 is schematic block diagram of the sleep monitoring device 120,
Figure 3 is schematic flow chart of a sleep monitoring method 300.

It should be noted that items which have the same reference numbers in different Figures, have the same structural features and the same functions, or are the same signals. Where the function and/or structure of such an item have been explained, there is no necessity for repeated explanation thereof in the detailed description.

### DETAILED DESCRIPTION OF EMBODIMENTS

While this invention is susceptible of embodiment in many different forms, there is shown in the drawings and will herein be described in detail one or more specific embodiments, with the understanding that the present disclosure is to be considered as exemplary of the principles of the invention and not intended to limit the invention to the specific embodiments shown and described.

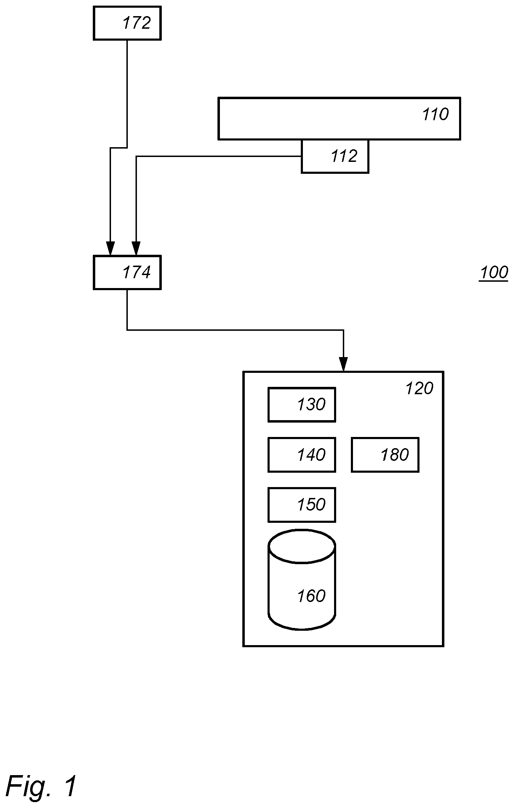

**Figure 1** is schematic block diagram of a sleep monitoring system 100. Sleep monitoring system 100 comprises a sleep monitoring device 120, a mattress 110, and a movement measurement device 112. Sleep monitoring device 120 is configured to monitor sleep states of a human, when the human is resting on mattress 110. Although the system may be applied to any human, the system is well suited to baby's. The illustrative embodiments shown below apply the system to babies; this does not mean that the system could not be applied to different age groups. The word baby means any child from birth to age 4. Newborn refers to babies under three months of age. Infants are babies from three months to one year old. Different sleep profiles apply to different age groups. For example, vocalization is irrelevant for adult sleep classification. Below we assume, the human is a baby.

In sleep science, the concept of sleep states is well established. Already, Prechtl, in his paper 'The behavioural states of the newborn infant (a review)' (Brain Research, 76, 1974, 185-212) defined a number of sleep states as well-defined conditions or properties that can be recognized if they occur again. Although different researches have subdivided the behavior pattern of humans while resting in more or fewer states, at least the following states are widely accepted as physiologically relevant: Quiet Sleep, Active Sleep, Quiet Alertness, Active Alertness, and Vocalization. Informally, these states range from fast asleep to drowsy, to crying. During a sleep episode, a baby will alternate through several of these stages, the so-called sleep cycle.

The development of the sleep-wake rhythm is dependent on the development of certain brain structures. It is known that in most cases the first manifestations of the start of the development of a day-night rhythm is around week 6 and is very definite after 12 weeks. During the development of a human from neonate to toddler, the sleep-wake rhythm develops from a feeding rhythm, to an established day-night rhythm.

Movement measurement device 112 is configured to obtain movement data of the human. Movement measurement device 112 may be implemented in a variety of ways, e.g., using an actimetry sensor worn by the human. Movement measurement device 112 is sufficiently sensitive to register gross as well as fine movement, in particular movement of the lungs and hearts. For example, this could be an actimetry sensor worn at chest level of the human body, as it allows for deriving hear rate and respiration data from the human. Preferably, the movement data also measures changes in body mass over time. It is preferred that movement measurement device 112 is non-invasive and in particular does not require any part of movement measurement device 112 to be in direct contact with the human.

In an embodiment, movement measurement device 112 comprises one or more load cells placed in or under mattress 110 for measuring pressure of the human on mattress 110. If multiple load cells are used, their signals may be combined in an improved signal. In an embodiment, movement measurement device 112 comprises a single load cell. The inventors have found that even with a single load cell sleep state classification is possible.

Interestingly, the load cell may also be used to determine the weight of the human from the load cell. In this way, sleep monitoring device 120 cannot only monitor a healthy development of sleep rhythm, but also of weight.

As an example, the load cell may be a piezo-electro touch sensor that can be placed under, say, a baby mattress to continuously measure the small movements of the baby required to detect heart beats.

Sleep monitoring device 120 may comprises a microphone 172 or be connectable to microphone 172 for recording vocalizations of the human. If movement measurement device 112 is sufficiently sensitive, a vocalization signal may also be determined from the movement signal based on spectral analyses.

Sleep monitoring device 120 may comprises an analog to digital converter 174 connected to movement measurement device 112 and microphone 172 (if present). ADC 174 may also be comprised in movement measurement device 112 and/or microphone 172 itself.

Sleep monitoring device 120 comprises a movement data analyzer 130 configured to compute from the movement data at least: heart beat data and respiration data. Additionally, movement data analyzer 130 may also compute gross movement data from the movement data. Gross movements are the large body movements of the human relating to the use of the large muscles of the human body, such as those in the legs, arms, and abdomen. Movement data analyzer 130 may split the movement data up into heart beat data, respiration data, e.g., by using frequency analysis; the typical frequency of heart rate is higher than that of respiration which in turn is higher than gross body movement: For babies up to 1 year, the resting heart rate lays between 100-160 bpm, for children aged 1-10 years old 60-140 bpm is considered healthy. Regarding respiration rate: For babies up to 6 weeks, the resting RR lays between 30-60 , for children aged up to 6 months old 25-40 is considered healthy and at the age of 3 this range is lowered to 20-30.

In an embodiment, the movement data is filtered with specific bandwidth filters to obtain heart beat data and respiration data. Movement may be based on the total energy is in the full signal, rather than a signal is a certain bandwidth.

Movement data analyzer 130 may also consider amplitudes; heart rate has a smaller amplitude than respiration which is smaller than body movement. Amplitude may be used to identify within the movement data whether it is hear rate, respiration rate or movement. In general it is true that the signal is most energetic and high amplitude, high frequency when it is body movement. The signal of respiration is of less amplitude and low frequency whereas the signal representing heart rate is of least amplitude, and thus is weakest but has a higher frequency as compared to respiratory.

Sleep monitoring device 120 comprises a data classifier 140 configured to determine a heart rate regularity valuation of the heart beat data within a time interval, and to determine a respiration regularity valuation of the respiration data within the time interval. In addition also hear rate may be obtained. Data classifier 140 also determines a movement extent indicating an extent of the gross and fine movement of the human within the time interval. Optionally, data classifier 140 also determines a vocalization extent indicating an extent of the vocalization within the time interval. Regularity valuations, gross and fine movement extent and vocalization extent are preferably scaled to the same scale, e.g., from -1 to +1 or from 0 to 1, etc.

Sleep monitoring device 120 comprises a sleep classifier 150 configured to obtain a sleep state for the time interval from at least the respiration regularity valuation, the heart rate regularity valuation and the gross and fine movement valuation. Many sleep states are characterized by the presence or absence of regularity in heart and/or respiration and/or gross and fine movement. By combining the regularity measures, a sleep state may be recognized.

Sleep monitoring device 120 may comprise a logging unit 160 for storing obtained sleep states. Sleep monitoring device 120 may also comprise a display unit for generating a display to show the stored sleep states visually. For example, the sleep states of last night may be indicate as a colored bar, in which a color indicates a particular state, and in which the bar indicates the progress of time. Data of multiple nights may be placed under each other, to make it easy to visually spot patterns.

Interestingly, sleep monitoring device 120 comprises a development classifier 180 configured to obtain a development state for the human from at least the logged sleep state data. For example, development classifier 180 may classify the sleep-wake rhythm of a particular night on a scale ranging from feeding rhythm, via emerging day-night rhythm, to an established day-night rhythm. The score may be included in the display, or made available for download, e.g., on a smart phone.

Based on the movement data the development of a baby in terms of sleep (i.e. the transformation from a feeding rhythm in newborns with frequent movements and wake moments in the nigh to a day night rhythm) and optionally the development of the weight of the baby may be determined. The development may be automatically logged by logging unit 160 and may be viewed by the parents using a smartphone or PC application or on device 120 itself.

Weight development may be logged in grams, decagrams, etc; sleep-rhythm development may be presented as development of movement counts over the night and/or the number of transitions between periods with movement and without movements. The more movement counts over the night, the more transitions between periods with movement and without movements, and the shorter the length of the periods with consecutive movement or non-movement, the less stable the sleep is. This can also be calculated as a fragmentation index. The development of the fragmentation index represents the sleep development.

The device may show movement counts, number of transitions, length of the periods with consecutive movement or non-movement, and/or fragmentation index with respect to the average baby of the same age and gender. The reference number may also be related to the age and gender of the newborn, e.g., obtained from a table stored in sleep monitoring device 120.

For example, development classifier 180 may comprise a weight table listing: gender and age, say in months, versus a reference weight. The reference weight may be a percentile distribution. For example, the table may give the weight at the 3, 5, 10, 25, 50, 75, 90, 95, 97 percentiles. The weight distribution may also represented by parameters, such as average and standard deviation.

Similar, to the weight table, development classifier 180 may comprise a sleep table, indicating a reference sleep-wake rhythm for a gender and age. In an embodiment, the age runs to 6 months, this is exemplary and may be more or less.

Through the weight and/or sleep-wake table development classifier 180 can report to the user if the sleep or weight development of the baby differs from average. If distribution information is available development classifier 180 may determine a variable that represent how far from the average the observed weight and sleep development is. For example, development classifier 180 may compute a percentile score.

Development classifier 180 may be configured with a receiver to receive age and gender of the baby.

Should health problems occur, in particular sleep or weight problems, then sleep or weight history records can be made available to, say, a pediatrician. Development classifier 180 may be used domestically even without medical supervision, in such case development classifier 180 should not be relied on for a medical diagnosis.

Thus using movement measurement device 112, e.g. a load cell or a piezo electro plate, a primary signal is collected. The primary signal can processed to obtain biomechanical, cardiovascular and respiratory data. In turn this data is processed into sleep and weight data. The latter step could be done online. Data may be presented to a user, say a parent, on a display, such as on a smartphone. In addition to sleep state classification the system may compute additional sleep data: sleep time, sleep state duration, and sleep fragmentation.

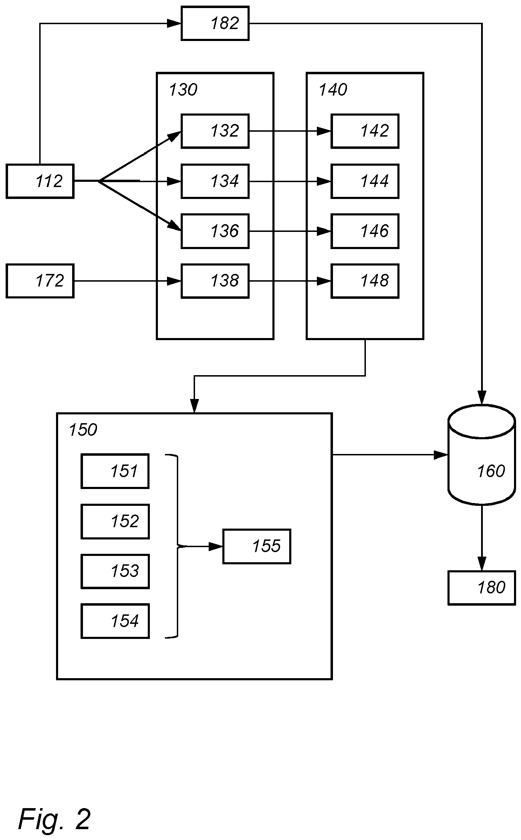

**Figure 2** is schematic block diagram of one possible embodiment of sleep monitoring device 120. Like in figure 1, sleep monitoring device 120 comprises movement data analyzer 130, data classifier 140, sleep classifier 150, logging unit 160, and development classifier 180.

Figure 2 further shows a weight determination unit 182 which may be part of sleep monitoring device 120. Weight determination unit 182 may be calibrated by placing the baby on mattress 110 and sending an externally determined weight of the baby to weight determination unit 182.

Movement data analyzer 130 comprises a heart beat unit 132, a respiration unit 134, a movement unit 136 and a vocalization unit 138.

Heart beat unit 132, respiration unit 134 and movement unit 136 are configured to compute from the movement data at least: heart beat data, respiration data, and gross and fine movement data. For example, heart beat unit 132, respiration unit 134 and movement unit 136 may comprise band filters that allow only signals to pass that have a frequency that is consistent with heart, respiration and movement respectively.

Movement data analyzer 130 may also comprise a frequency analyzer for computing a spectrum of the movement data, for example, a Fourier or wavelet transform. Heart beat unit 132, respiration unit 134 and movement unit 136 may then select the coefficients that correspond to their particular frequencies. Gross movement data may also be defined as the part of the signal that is left after subtraction of the heart and respiration data.

Alternatively, or in addition, the amplitude may be used, to split the movement data in these three parts. Heart data correspond to local smaller and faster fluctuations in the signal, respiration to larger and slower and less local fluctuations.

However, preferably, the movement unit 146 does not use a filter, or only a very broad filter. Movement information may be derived from amplitude variations and the amount of energy in the signal. Gross movement may impair the extraction of heart beat data and respiration data. This may be resolved by ignoring this data when it is not available. When movement is absent amplitude of the signal related to the respiration is of relative large amplitude as compared to the signal part reflecting heart beat.

Vocalization unit 138 may take the audio signal of microphone 172 as is, but preferably a band pass filter is applied to filter the frequency of human babies crying. The filter may be relatively small compared to filters used for movement data, there is little information loss if part of the spectrum of a crying sound is lost, and on the other hand a tight filter may reduce false positives considerably.

Data classifier 140 is configured to classify the signals produced by movement data analyzer 130 in a specified time interval. A traditional time interval is 3 minutes, shorter time intervals, say 30 seconds, or longer say 5 minutes are possible. The classification may be done on running time intervals; this means that a classification will be obtained for consecutive instances, say each second. The classification may be done on consecutive time intervals; this means that a classification will be obtained each time the time interval elapsed. Data classifier 140 need only be operative when sleep classification is required, e.g., so long as a signal is obtained from movement measurement device 112. Data classifier 140 may also have an input for the user to indicate that a human is currently resting on mattress 110.

Based on the data of movement data analyzer 130 it is classified by data classifier 140 if the heart rate and respiration rate are regular, if the movement can be classified a light movement, gross movement, i.e., as a value indicating the extent of the movement, and the vocalization.

Data classifier 140 comprises a heart beat classifier 142, a respiration classifier 144, a movement classifier 146, a vocalization classifier 148.

Heart beat classifier 142 and respiration classifier 144 compute regularity valuation for the signal within the time interval; heart beat classifier 142 computes a heart rate regularity valuation of the heart beat data within a time interval, respiration classifier 144 computes a respiration regularity valuation of the respiration data within the time interval. Preferably, Heart beat classifier 142 and respiration classifier 144 also determine the heart and respiration rate.

Computing regularity data may be implemented in a number of ways. Fourier analysis may be used. A particular well suited way is to determine peaks in the correlogram, also referred to as the autocorrelation function. For example, the height of the third peak in the correlogram (counting the peak at lag 0 as the first peak) indicates the rhythmicity of the signal. However, the art has many other measures for heart rate variability which may be applied, to the heart beat data and analogously to the respiration data. A more variability measure is obtained as the square root of the mean squared difference between adjacent N-N intervals. (RMSSD). The N-N interval is the time between 2 heartbeats. In an embodiment, the RMSSD is calculated per classifier interval, e.g. ranging from 30 sec to 5 min.

Movement classifier 146 is configured to determine a gross movement extent indicating an extent of the gross movement of the human within the time interval and a fine movement extent. Vocalization classifier 148 is configured to determine a vocalization extent indicating an extent of the vocalization within the time interval. For example, movement classifier 146 to obtain gross movement extent. Vocalization classifier 148 may be configured to integrate their respective input signal, or to compute a maximum amplitude thereof.

Vocalization classifier 148 may be configured to map the vocalization to an S-curve, e.g., so that vocalization above a threshold will give the maximum value.

The heart rate regularity valuation, the respiration regularity valuation, movement extent and/or vocalization extent are further processed by sleep classifier 150. Also the rate may be processed, both for heart and respiration. Sleep classifier 150 preferably scales these values, e.g., so that they all lie in the same range, e.g., from -1 to +1, or 0 to 1, etc.

Sleep classifier 150 comprises data that characterize a sleep state. Sleep classifier 150 comprises a matching unit 155 which matches the classified data obtained from data classifier 140 to the sleep state characterizing data. The latter may be advantageously represented by storing a classifying vector associated with a sleep state. Figure 2 shows vectors 151, 152, 153, 154, there may be more or less.
The table below shows an example of representing sleep state using vectors:

| *State* | Regular Heart Rate | Regular Respiration | Gross Movement | Fine Movement | Vocalization |
|---|---|---|---|---|---|
| Quiet Sleep | +1 | +1 | -1 | -1 | -1 |
| Active Sleep | -1 | -1 | -1 | +1 | -1 |
| Quiet Alertness | +1 | +1 | -1 | +1 | -1 |
| Active Alertness | -1 | -1 | +1 | +1 | -1 |
| Vocalization | | -1 | +1 | +1 | +1 |

In this example 5 sleep/wake states are represented with 5 vectors, each having 5 components. -1 denotes absence, +1 presence and 0 indifference of a feature. (In this case the vectors are shown as row vectors). This table defines sleep states in terms of the regularity of heart rate and respiration, the extent of gross and fine movement and amount of vocalization. This differs from the classic definition, (see e.g., Prechtl), which uses eye closure status. Experiments have shown that the classification obtained from the definition above correlates with the classic definition of Prechtl. Sleep monitoring device 120 can determine a classification according to the table above completely from contactless sensing means, e.g., load cells and/or microphones.

In an embodiment, a matching value is calculated between the classified data vector and a classifying vector associated with a sleep state. The matching value may be an inner product, a correlation coefficient, a rank order correlation, or the like. Below we assume inner product, which also performs well for continuous features. For feature values one may also count the number of equal vector elements with the stored characterizing data.

One way for matching unit 155 to match the classified data of data classifier 140 with the data stored in sleep classifier 150 is to represent the classified data as a vector as well, i.e., a classified data vector comprising as elements at least the heart rate regularity valuation, the respiration regularity valuation, and preferably, gross movement extent and vocalization extent. The inner product between the classified data vector and a classifying vector associated with a sleep state. The result of the inner product is a measure for how close the current observed data matches the characterizing sleep data.

This procedure may be repeated for all vectors stored in sleep classifier 150, i.e., when using the table above this will produce 5 values corresponding to the 5 sleep states. There are different ways to proceed with these 5 values. In one embodiment, a sleep state may be selected as the obtained sleep state, i.e., the final classified sleep state, if the matching data, i.e., the inner product matches a threshold or range associated with the particular sleep state. For example, if an inner product is at least 1.5 the sleep state is present. This has the result that sometimes, e.g., during transitions, multiple sleep states may be obtained. Although reporting multiple sleep states may do more justice to reality, it may be very confusing for the user.

Alternatively, the classified data vector is a feature vector, the elements of which only indicate the presence or absence of feature. The classified data vector may be compared with the classifying vector. If this profile is met, then the state is presented. If no profile is met, then one could keep the last state as the current state or presenting the state most proximate to the obtained profile or presenting a non-determined state.

In an embodiment, the multiple inner products are combined into a single color, which in turn can be shown on a bar, say as a, hypnogram. This has the advantage that transitions from one phase to a next phase, will be shown with a transition of colors.

In an embodiment, sleep classifier 150 is configured to select the sleep state associate with a largest inner product of the multiple inner products as the obtained sleep state. This has the advantage of being well defined in all circumstances.

Alternatively, or in addition data classifier 140 may determine: movement counts, e.g., the amount of movement above a certain threshold; Heart rate, e.g., balistocardiography in beats per minute; Respiration rate, e.g., in breathings per minute. This data can be interpreted in terms of sleep stability and sleep. These values may also be matched by sleep classifier 150 to characterizing data to obtain finer recognition of sleep states. Alternatively, one or more of these values may be compared by development classifier 180 to reference values to report on development of the baby in this regard.

Collected movement data may thus also be interpreted as movement, heart rate, and as breathing rate. This heart rate, breathing rate and movement data can also be interpreted in terms of sleep. This may distinguish just wake from sleep, but also differentiate between "lighter" sleep and deeper sleep, or consistent versus irregular sleep.

Sleep states may be presented as percentages of the night. They may even be merged into a single number if needed.

For example, sleep monitoring device 120 may be configured to also determine the sleep stability in addition to sleep state. Sleep stability is derived from the movement counts overnight. The more movement count over the night and the more transitions between periods with movement and without movements, the less stable the sleep is. These values may be compared with reference values for age and gender of the baby, to give an indication of normality (close to the average) or abnormality (away from average) of the sleep behavior.

This can be interpreted in terms of development: When newborns reflect less stable sleep, they are considered to be in a feeding driven sleep-wake behavior, rather than a day-night sleep-wake rhythm.

Sleep monitoring device 120 further comprises a weight determination unit 182 to determine the weight from the movement data. Weight is dependent on the amplitude of the movement data, when the baby is in rest. The higher the amplitude, the more the baby is weighting. Weight can be presented in grams, rounded in decagrams. Data can be compared to regular baby developmental weight for age tables, in development classifier 180.

Development classifier 180 may also use heart and respiration data to check the cardiovascular state of the baby. Measured heart rate and respiration rate can be checked against the standard for children in these age groups: For babies up to 1 year, the resting heart rate lays between 100-160 bpm, for children aged 1-10 years old 60-140 bpm is considered healthy. Regarding respiration rate: For babies up to 6 weeks, the resting respiration rate lies between 30-60, for children aged up to 6 months old 25-40 is considered healthy and at the age of 3 this range is lowered to 20-30.

Typically, the device 120 comprises a microprocessor (not shown) which executes appropriate software stored at the device 120; for example, that software may have been downloaded and/or stored in a corresponding memory, e.g., a volatile memory such as RAM or a non-volatile memory such as Flash (not shown). Alternatively, the devices 120 may, wholly or partially, be implemented in programmable logic, e.g., as field-programmable gate array (FPGA).

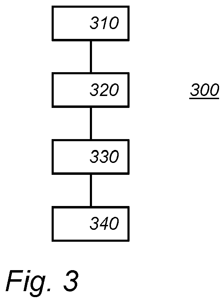

Figure 3 shows a schematic flowchart 300 of a sleep monitoring method for monitoring sleep states of a human. The sleep monitoring method comprising: obtaining 310 movement data of the human, computing 320 from the movement data at least: heart beat data, and respiration data, determining 330 a heart rate regularity valuation of the heart beat data within a time interval, and determining a respiration regularity valuation of the respiration data within the time interval, and obtaining 340 a sleep state for the time interval from at least the respiration regularity valuation, the heart rate regularity valuation and the motion.

Many different ways of executing the method are possible, as will be apparent to a person skilled in the art. For example, the order of the steps can be varied or some steps may be executed in parallel. Moreover, in between steps other method steps may be inserted. The inserted steps may represent refinements of the method such as described herein, or may be unrelated to the method. For example, a given step may not have finished completely before a next step is started.

A method according to the invention may be executed using software, which comprises instructions for causing a processor system to perform method 300. Software may only include those steps taken by a particular sub-entity of the system. The software may be stored in a suitable storage medium, such as a hard disk, a floppy, a memory, in the cloud, etc. The software may be sent as a signal along a wire, or wireless, or using a data network, e.g., the Internet. The software may be made available for download and/or for remote usage on a server. A method according to the invention may be executed using a bitstream arranged to configure programmable logic, e.g., a field-programmable gate array (FPGA), to perform a method according to the invention.

It will be appreciated that the invention also extends to computer programs, particularly computer programs on or in a carrier, adapted for putting the invention into practice. The program may be in the form of source code, object code, a code intermediate source and object code such as partially compiled form, or in any other form suitable for use in the implementation of the method according to the invention. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the means of at least one of the systems and/or products set forth.

It should be noted that the above-mentioned embodiments illustrate rather than limit the invention, and that those skilled in the art will be able to design many alternative embodiments.

In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A sleep monitoring device (120) for monitoring sleep states of a human, the sleep monitoring device is configured to receive movement data of the resting human from a movement measurement device (112), the sleep monitoring device (120) comprises
- a movement data analyzer (130) configured to compute from the movement data at least: heart beat data, respiration data, gross movement data, and fine movement data,
- a data classifier (140) configured to determine a heart rate regularity valuation of the heart beat data within a time interval, to determine a respiration regularity valuation of the respiration data within the time interval, to determine a gross movement extent indicating an extent of the gross movement of the human within the time interval, and to determine a fine movement extent indicating an extent of the fine movement of the human within the time interval, wherein the data classifier (140) is configured to compute a movement extent from the movement data, if the movement extent is over a first threshold, then gross movement is determined, if the movement extent is over a second threshold but not over the first threshold then fine movement is present, the second threshold being smaller than the first threshold, if the movement extent is below the second threshold then neither fine nor gross movement is determined, and
- a sleep classifier (150) configured to obtain a sleep state for the time interval from at least the respiration regularity valuation, the heart rate regularity valuation, the gross movement extent, and the fine movement extent.

2. A sleep monitoring device (120) as in Claim 1, wherein the data classifier (140) is configured to subtract the heart beat date and the respiration data from the movement data.

3. A sleep monitoring device (120) as in Claim 2, wherein the computed movement extent is total energy, largest amplitude, or largest amplitude difference.

4. A sleep monitoring device as in any one of the preceding claims, wherein the sleep monitoring device further comprises
- a microphone (172) for recording vocalizations of the human, and
- a vocalization unit (138) configured to apply a band pass filter to an audio signal of microphone (172), the bandpass filter having a passband arranged to pass a frequency of human babies crying,
- and wherein the data classifier is configured to determine a vocalization extent indicating an extent of the vocalization within the time interval.

5. A sleep monitoring device as in any one of the preceding claims wherein the sleep classifier is configured to determine a sleep state by
- determining a classified data vector comprising as elements at least the heart rate regularity valuation, the respiration regularity valuation, gross movement extent, fine movement extent and/or vocalization extent,
- calculating an matching value between the classified data vector and a classifying vector associated with a sleep state.

6. A sleep monitoring device as in Claim 5 wherein the sleep classifier is configured to
- calculate multiple matching value between the classified data vector and one of multiple classifying vectors associated with multiple sleep states,
- select the sleep state associated with a largest matching value of the multiple matching value as the obtained sleep state.

7. A sleep monitoring device as in Claim 5 wherein the sleep classifier is configured to select the associated sleep state as the obtained sleep state if the matching value is within a threshold or range associated with the particular sleep state.

8. A sleep monitoring device as in any one of the preceding claims, wherein
- determining the heart rate regularity valuation comprises determining the Root Mean Square of the Successive Differences of the heart beat data, and/or
- determining the respiration regularity valuation comprises determining the Root Mean Square of the Successive Differences of the respiration data.

9. A sleep monitoring device as in any one of the preceding claims 1, wherein the movement measurement device comprises a load cell for measuring pressure of the human on a mattress on which the human rests.

10. A sleep monitoring device as in any one of the preceding claims comprising a logging unit (160) for storing obtained sleep states.

11. A sleep monitoring device as in Claim 10 comprising a development classifier (180) configured to obtain a development state for the human from at least the logged sleep state data.

12. A sleep monitoring system comprising the sleep monitoring device as in any one of the preceding claims, a mattress and the movement measurement device arranged with respect to the mattress to measure movement data representing movement of a resting human on the mattress.

13. A sleep monitoring method for monitoring sleep states of a human, the sleep monitoring method comprising
- obtaining (310) movement data of the human,
- computing (320) from the movement data at least: heart beat data, respiration data, and gross movement data, and fine movement data,
- determining (330) a heart rate regularity valuation of the heart beat data within a time interval, determining a respiration regularity valuation of the respiration data within the time interval, to determine a gross movement extent indicating an extent of the gross movement of the human within the time interval, and to determine a fine movement extent indicating an extent of the fine movement of the human within the time interval, and computing a movement extent from the movement data, if the movement extent is over a first threshold, then gross movement is determined, if the movement extent is over a second threshold but not over the first threshold then fine movement is present, the second threshold being smaller than the first threshold, if the movement extent is below the second threshold then neither fine nor gross movement is determined, and
- obtaining (340) a sleep state for the time interval from at least the respiration regularity valuation, the heart rate regularity valuation, the gross movement extent, and the fine movement extent.

14. A computer program comprising computer program code means adapted to perform all the steps of claim 13 when the computer program is run on a computer.

15. A computer program as claimed in claim 14 embodied on a computer readable medium.

## Patentansprüche

1. Schlafüberwachungsvorrichtung (120) zur Überwachung von Schlafzuständen eines Menschen, wobei die Schlafüberwachungsvorrichtung dafür konfiguriert ist, Bewegungsdaten des ruhenden Menschen von einer Bewegungsmessvorrichtung (112) zu empfangen, wobei die Schlafüberwachungsvorrichtung (120) Folgendes umfasst:
- einen Bewegungsdatenanalysator (130), der dafür konfiguriert ist, anhand der Bewegungsdaten mindestens Herzschlagdaten, Atmungsdaten, Grobbewegungsdaten und Feinbewegungsdaten zu berechnen,
- einen Datenklassifizierer (140), der dafür konfiguriert ist, eine Herzfrequenzregelmäßigkeitsbewertung der Herzschlagdaten innerhalb eines Zeitintervalls zu ermitteln, eine Atmungsregelmäßigkeitsbewertung der Atmungsdaten innerhalb des Zeitintervalls zu ermitteln, ein Grobbewegungsausmaß, das ein Ausmaß der Grobbewegung des Menschen angibt, innerhalb des Zeitintervalls zu ermitteln, und ein Feinbewegungsausmaß, das ein Ausmaß einer Feinbewegung des Menschen angibt, innerhalb der Zeitintervalls zu ermitteln, wobei der Datenklassifizierer (140) dafür konfiguriert ist, ein Bewegungsausmaß anhand der Bewegungsdaten zu berechnen, und wenn das Bewegungsausmaß über einem ersten Schwellenwert liegt, wird Grobbewegung festgestellt, wenn das Bewegungsausmaß über einem zweiten Schwellenwert liegt, aber nicht über dem ersten Schwellenwert liegt, wobei der zweite Schwellenwert kleiner als der erste Schwellenwert ist, liegt Feinbewegung vor, wenn das Bewegungsausmaß unter dem zweiten Schwellenwert liegt, wird weder Fein- noch Grobbewegung festgestellt, und
- einen Schlafklassifizierer (150), der dafür konfiguriert ist, einen Schlafzustand für das Zeitintervall anhand von mindestens der Atmungsregelmäßigkeitsbewertung, der Herzfrequenzregelmäßigkeitsbewertung, des Grobbewegungsausmaßes und des Feinbewegungsausmaßes zu erlangen.

2. Schlafüberwachungsvorrichtung (120) nach Anspruch 1, wobei der Datenklassifizierer (140) dafür konfiguriert ist, die Herzschlagdaten und die Atmungsdaten von den Bewegungsdaten zu subtrahieren.

3. Schlafüberwachungsvorrichtung (120) nach Anspruch 2, wobei das berechnete Bewegungsausmaß die Gesamtenergie, die größte Amplitude oder die größte Amplitudendifferenz ist.

4. Schlafüberwachungsvorrichtung nach einem der vorhergehenden Ansprüche, wobei die Schlafüberwachungsvorrichtung weiterhin Folgendes umfasst:
- ein Mikrofon (172) zum Aufzeichnen von Vokalisierungen des Menschen, und
- eine Vokalisierungseinheit (138), die dafür konfiguriert ist, einen Bandpassfilter auf ein Audiosignal des Mikrofons (172) anzuwenden, wobei der Bandpassfilter ein Durchlassband hat, das dafür ausgelegt ist, eine Frequenz der Schreilaute von menschlichen Säuglingen durchzulassen,
- und wobei der Datenklassifizierer dafür konfiguriert ist, ein Vokalisierungsausmaß zu ermitteln, das ein Ausmaß der Vokalisierung innerhalb des Zeitintervalls angibt.

5. Schlafüberwachungsvorrichtung nach einem der vorhergehenden Ansprüche, wobei der Schlafklassifizierer dafür konfiguriert ist, einen Schlafzustand zu ermitteln durch
- Ermitteln eines klassifizierten Datenvektors, der als Elemente mindestens die Herzfrequenzregelmäßigkeitsbewertung, die Atmungsregelmäßigkeitsbewertung, Grobbewegungsausmaß, Feinbewegungsausmaß und/oder Vokalisierungsausmaß umfasst,
- Berechnen eines Übereinstimmungswerts zwischen dem klassifizierten Datenvektor und einem zu einem Schlafzustand gehörigen Klassifizierungsvektor.

6. Schlafüberwachungsvorrichtung nach Anspruch 5, wobei der Schlafklassifizierer konfiguriert ist zum
- Berechnen mehrerer Übereinstimmungswerte zwischen dem klassifizierten Datenvektor und einem von mehreren zu mehreren Schlafzuständen gehörigen Klassifizierungsvektoren,
- Auswählen des zu einem größten Übereinstimmungswert von den mehreren Übereinstimmungswerten gehörigen Schlafzustands als den erlangten Schlafzustand.

7. Schlafüberwachungsvorrichtung nach Anspruch 5, wobei der Schlafklassifizierer dafür konfiguriert ist, den zugehörigen Schlafzustand als den erlangten Schlafzustand auszuwählen, wenn der Übereinstimmungswert innerhalb eines zu dem betreffenden Schafzustand gehörigen Schwellenwerts oder Bereichs liegt.

8. Schlafüberwachungsvorrichtung nach einem der vorhergehenden Ansprüche, wobei
- das Ermitteln der Herzfrequenzregelmäßigkeitsbewertung das Ermitteln des Effektivwerts von aufeinanderfolgenden Differenzen der Herzschlagdaten umfasst, und/oder
- das Ermitteln der Atmungsregelmäßigkeitsbewertung das Ermitteln des Effektivwerts von aufeinanderfolgenden Differenzen der Atmungsdaten umfasst.

9. Schlafüberwachungsvorrichtung nach einem der vorhergehenden Ansprüche 1 , wobei die Bewegungsmessvorrichtung eine Druckmessdose zum Messen des Drucks des Menschen auf eine Matratze umfasst, auf welcher der Mensch liegt.

10. Schlafüberwachungsvorrichtung nach einem der vorhergehenden Ansprüche, umfassend eine Protokollierungseinheit (160) zum Speichern von erlangten Schlafzuständen.

11. Schlafüberwachungsvorrichtung nach Anspruch 10, umfassend einen Entwicklungsklassifizierer (180), der dafür konfiguriert ist, einen Entwicklungszustand für den Menschen anhand von mindestens den protokollierten Schlafzustandsdaten zu erlangen.

12. Schlafüberwachungssystem umfassend die Schlafüberwachungsvorrichtung nach einem der vorhergehenden Ansprüche, eine Matratze und die Bewegungsmessvorrichtung, die in Bezug auf die Matratze angeordnet ist, um Bewegungsdaten zu messen, die Bewegungen eines auf der Matratze liegenden Menschen darstellen.

13. Schlafüberwachungsverfahren zum Überwachen von Schlafzuständen eines Menschen, wobei das Schlafüberwachungsverfahren Folgendes umfasst:
- Erlangen (310) von Bewegungsdaten des Menschen,
- Berechnen (320) von mindestens Herzschlagdaten, Atmungsdaten, Grobbewegungsdaten und Feinbewegungsdaten anhand der Bewegungsdaten,
- Ermitteln (330) einer Herzfrequenzregelmäßigkeitsbewertung der Herzschlagdaten innerhalb eines Zeitintervalls, Ermitteln einer Atmungsregelmäßigkeitsbewertung der Atmungsdaten innerhalb des Zeitintervalls, Ermitteln eines Grobbewegungsausmaßes, das ein Ausmaß der Grobbewegung des Menschen angibt, innerhalb des Zeitintervalls, und Ermitteln eines Feinbewegungsausmaß, das ein Ausmaß einer Feinbewegung des Menschen angibt, innerhalb der Zeitintervalls, und Berechnen eines Bewegungsausmaßes anhand der Bewegungsdaten, und wenn das Bewegungsausmaß über einem ersten Schwellenwert liegt, wird Grobbewegung festgestellt, wenn das Bewegungsausmaß über einem zweiten Schwellenwert liegt, aber nicht über dem ersten Schwellenwert liegt, wobei der zweite Schwellenwert kleiner als der erste Schwellenwert ist, liegt Feinbewegung vor, wenn das Bewegungsausmaß unter dem zweiten Schwellenwert liegt, wird weder Fein- noch Grobbewegung festgestellt, und
- Erlangen (340) eines Schlafzustands für das Zeitintervall anhand von mindestens der Atmungsregelmäßigkeitsbewertung, der Herzfrequenzregelmäßigkeitsbewertung, des Grobbewegungsausmaßes und des Feinbewegungsausmaßes.

14. Computerprogramm umfassend Computerprogrammcodemittel, die dafür ausgelegt sind, alle Schritte von Anspruch 13 durchzuführen, wenn das Computerprogramm auf einem Computer ausgeführt wird.

15. Computerprogramm nach Anspruch 14, verkörpert auf einem computerlesbaren Medium.

## Revendications

1. Dispositif de surveillance de sommeil (120) permettant de surveiller des états de sommeil d'un humain, le dispositif de surveillance de sommeil étant configuré pour recevoir des données de mouvement de l'humain au repos à partir d'un dispositif de mesure de mouvement (112), le dispositif de surveillance de sommeil (120) comprenant
- un analyseur de données de mouvement (130) configuré pour calculer à partir des données de mouvement au moins : des données de battements de coeur, des données de respiration, des données de mouvement grossier et des données de mouvement fin,
- un classificateur de données (140) configuré pour déterminer une évaluation de régularité de fréquence cardiaque des données de battements de coeur au cours d'un intervalle de temps, pour déterminer une évaluation de régularité de respiration des données de respiration au cours de l'intervalle de temps, pour déterminer une étendue de mouvement grossier indiquant une étendue du mouvement grossier de l'humain au cours de l'intervalle de temps, et pour déterminer une étendue de mouvement fin indiquant une étendue du mouvement fin de l'humain au cours de l'intervalle de temps, dans lequel le classificateur de données (140) est configuré pour calculer une étendue de mouvement à partir des données de mouvement, si l'étendue de mouvement est au-dessus d'un premier seuil, alors un mouvement grossier est déterminé, si l'étendue de mouvement est au-dessus d'un second seuil mais pas au-dessus du premier seuil alors un mouvement fin est présent, le second seuil étant plus petit que le premier seuil, si l'étendue de mouvement est en dessous du second seuil alors ni un mouvement fin ni un mouvement grossier ne sont déterminés, et
- un classificateur de sommeil (150) configuré pour obtenir un état de sommeil pour l'intervalle de temps à partir au moins de l'évaluation de régularité de respiration, l'évaluation de régularité de fréquence cardiaque, l'étendue de mouvement grossier et l'étendue de mouvement fin.

2. Dispositif de surveillance de sommeil (120) selon la revendication 1, dans lequel le classificateur de données (140) est configuré pour soustraire les données de battements de coeur et les données de respiration des données de mouvement.

3. Dispositif de surveillance de sommeil (120) selon la revendication 2, dans lequel l'étendue de mouvement calculée est une énergie totale, la plus grande amplitude ou la plus grande différence d'amplitude.

4. Dispositif de surveillance de sommeil selon l'une quelconque des revendications précédentes, dans lequel le dispositif de surveillance de sommeil comprend en outre
- un microphone (172) permettant d'enregistrer des vocalisations de l'humain, et
- une unité de vocalisation (138) configurée pour appliquer un filtre passe-bande à un signal audio du microphone (172), le filtre passe-bande ayant une bande passante agencée pour laisser passer une fréquence de bébés humains pleurant,
- et dans lequel le classificateur de données est configuré pour déterminer une étendue de vocalisation indiquant une étendue de la vocalisation au cours de l'intervalle de temps.

5. Dispositif de surveillance de sommeil selon l'une quelconque des revendications précédentes dans lequel le classificateur de sommeil est configuré pour déterminer un état de sommeil en
- déterminant un vecteur de données classifiées comprenant comme éléments au moins l'évaluation de régularité de fréquence cardiaque, l'évaluation de régularité de respiration, l'étendue de mouvement grossier, l'étendue de mouvement fin et/ou l'étendue de vocalisation,
- calculant une valeur de correspondance entre le vecteur de données classifiées et un vecteur de classification associé à un état de sommeil.

6. Dispositif de surveillance de sommeil selon la revendication 5 dans lequel le classificateur de sommeil est configuré pour
- calculer une valeur de correspondance multiple entre le vecteur de données classifiées et l'un de multiples vecteurs de classification associés à de multiples états de sommeil,
- sélectionner l'état de sommeil associé à la plus grande valeur de correspondance de la valeur de correspondance multiple comme état de sommeil obtenu.

7. Dispositif de surveillance de sommeil selon la revendication 5 dans lequel le classificateur de sommeil est configuré pour sélectionner l'état de sommeil associé comme état de sommeil obtenu si la valeur de correspondance s'inscrit dans un seuil ou une gamme associés à l'état de sommeil particulier.

8. Dispositif de surveillance de sommeil selon l'une quelconque des revendications précédentes, dans lequel
- la détermination de l'évaluation de la régularité de fréquence cardiaque comprend la détermination de la moyenne quadratique des différences successives des données de battements de coeur, et/ou
- la détermination de l'évaluation de régularité de respiration comprend la détermination de la moyenne quadratique des différences successives des données de respiration.

9. Dispositif de surveillance de sommeil selon l'une quelconque des revendications précédentes 1, dans lequel le dispositif de mesure de mouvement comprend une cellule de mesure permettant de mesurer une pression de l'humain sur un matelas sur lequel l'humain se repose.

10. Dispositif de surveillance de sommeil selon l'une quelconque des revendications précédentes comprenant une unité de journalisation (160) permettant de stocker des états de sommeil obtenus.

11. Dispositif de surveillance de sommeil selon la revendication 10 comprenant un classificateur de développement (180) configuré pour obtenir un état de développement pour l'humain à partir au moins des données d'état de sommeil journalisées.

12. Système de surveillance de sommeil comprenant le dispositif de surveillance de sommeil selon l'une quelconque des revendications précédentes, un matelas et le dispositif de mesure de mouvement agencé par rapport au matelas pour mesurer des données de mouvement représentant un mouvement d'un humain se reposant sur le matelas.

13. Procédé de surveillance de sommeil permettant de surveiller des états de sommeil d'un humain, le procédé de surveillance de sommeil comprenant
- l'obtention (310) de données de mouvement de l'humain,
- le calcul (320) à partir des données de mouvement d'au moins : des données de battements de coeur, des données de respiration et des données de mouvement grossier et des données de mouvement fin,
- la détermination (330) d'une évaluation de régularité de fréquence cardiaque des données de battements de coeur au cours d'un intervalle de temps, la détermination d'une évaluation de régularité de respiration des données de respiration au cours de l'intervalle de temps, pour déterminer une étendue de mouvement grossier indiquant une étendue du mouvement grossier de l'humain au cours de l'intervalle de temps, et pour déterminer une étendue de mouvement fin indiquant une étendue du mouvement fin de l'humain au cours de l'intervalle de temps, et le calcul d'une étendue de mouvement à partir des données de mouvement, si l'étendue de mouvement est au-dessus d'un premier seuil, alors un mouvement grossier est déterminé, si l'étendue de mouvement est au-dessus d'un second seuil mais pas au-dessus du premier seuil alors un mouvement fin est présent, le second seuil étant plus petit que le premier seuil, si l'étendue de mouvement est en dessous du second seuil alors ni un mouvement fin ni un mouvement grossier ne sont déterminés, et
- l'obtention (340) d'un état de sommeil pour l'intervalle de temps à partir au moins de l'évaluation de régularité de respiration, l'évaluation de régularité de fréquence cardiaque, l'étendue de mouvement grossier et l'étendue de mouvement fin.

14. Programme d'ordinateur comprenant des moyens de code de programme d'ordinateur adaptés pour mettre en oeuvre toutes les étapes de la revendication 13 lorsque le programme d'ordinateur tourne sur un ordinateur.

15. Programme d'ordinateur selon la revendication 14 inclus sur un support lisible par ordinateur.
